# DEMANDE DE BREVET EUROPEEN

(11) **EP 0 771 552 A1**
(43) Date de publication de la demande: **07.05.1997**
(21) Numéro de dépôt: 96402332.9
(22) Date de dépôt: 31.10.1996
(51) Int. Cl.: A61F 2/34

(54) **Cupule non vissée à implanter sans ciment et prothèse cotyloidienne associée**

(30) Priorité: 03.11.1995 FR 9513016
(71) Demandeur: Negre, Jacques Etienne Léopold, Dr., 45000 Orléans (FR); Henry, François Xavier Louis, Dr., 45800 Saint-Jean-de-Braye (FR)
(72) Inventeur: Negre, Jacques Etienne Léopold, Dr., 45000 Orléans (FR); Henry, François Xavier Louis, Dr., 45800 Saint-Jean-de-Braye (FR)
(74) Mandataire: Beauchamps, Georges

(57) **Abrégé**

La présente invention concerne une cupule non vissée et non cimentée.

La cupule (1) de l'invention comporte une tige de guidage allongée (6) destinée à faire saillie de la surface externe (la) de la cupule, non filetée sur toute son étendue extérieurement saillante et destinée à être reçue dans un trou percé dans la cavité cotyloïdienne sur une longueur suffisante pour permettre l'enfoncement de la tige dans celui-ci lors de la migration de la cupule dans le bassin au cours de la consolidation.

L'invention s'applique à la chirurgie de première intention et de reprise.

## Description

La présente invention concerne une cupule non cimentée et non vissée de première intention ou de reprise à implanter dans la cavité cotyloïdienne osseuse, et la prothèse cotyloïdienne associée.

En cas d'échec d'une chirurgie de première intention, par exemple en cas de descellement cotyloïdien, il est nécessaire de réopérer le patient pour retirer l'ancienne prothèse et implanter une cupule de reprise dans la cavité.

Le descellement de la cupule de première intention peut se produire aussi bien dans les poses avec ciment, lorsque la prise du ciment est défectueuse, que sans ciment, lorsque l'ancrage primaire et/ou secondaire n'assure pas une stabilité suffisante de l'implant.

Dans les deux cas, il est nécessaire de nettoyer la cavité cotyloïdienne avant l'implantation de la cupule de reprise, ce qui résulte en une cavité élargie où l'os est en quantité moindre.

Il faut alors réépaissir l'os en apportant des greffons, tels que des fragments d'os spongieux, dans la cavité cotyloïdienne en vue de la reconstruction osseuse.

La cupule de reprise vient alors coiffer l'ensemble des greffons et de la cavité cotyloïdienne nettoyée.

Lorsque le cotyle présente des malformations, il peut également être nécessaire d'ajouter de tels greffons dès la chirurgie de première intention.

Au cours de la consolidation qui dure au moins deux mois, il se produit un tassement de ces greffons sous l'effet de la pression exercée par la cupule sur le toit du cotyle osseux.

Ce phénomène de tassement des greffons engendre de nombreux problèmes dans les cupules actuellement connues qui sont équipées de vis pour l'ancrage primaire dans le cotyle.

En effet, ces cupules ne peuvent s'enfoncer dans la cavité pour suivre le tassement des greffons si des vis sont implantées à demeure dans l'os, ce qui limite le réépaississement de l'os par tassement des greffons et donc leur consolidation.

S'il y a néanmoins une migration de la cupule dans la cavité cotyloïdienne, les vis viennent buter sur l'insert en polyéthylène, ce qui engendre des micro-déplacements ou des instabilités de position de la cupule dans la cavité. Les frottements entre les têtes de vis et l'insert risquent en outre de détacher des particules en polyéthylène provoquant une résorption osseuse par réaction à ces corps étrangers libérés dans le cotyle.

D'autre part, les vis utilisées pour l'ancrage primaire sont généralement recouvertes distalement d'hydroxyapatite pour la réhabitation osseuse, ce qui engendre une opacification de l'os autour des pas de vis, puis une résorption osseuse sous l'action des contraintes transmises par les vis.

Au surplus, avec les cupules vissées et impactées du type "press fit", la stabilité biologique à long terme de l'implant dans le cotyle n'est pas très fiable en raison d'une mauvaise répartition des pressions exercées par la cupule sur le toit du cotyle osseux.

En effet, l'os iliaque est très élastique et comporte des zones osseuses plus ou moins molles faisant varier les forces de vissage dans le cotyle. Il en résulte des moments de torsion provoquant l'arrachement ou le décollement de certaines vis par rapport aux autres, ce qui nuit à la stabilité biologique de l'ensemble.

La présente invention a donc pour but d'éliminer les inconvénients précités et de proposer une cupule non cimentée et non vissée qui puisse servir en première intention ou pour une reprise, qui augmente la stabilité biologique de la cupule dans le cotyle et qui permette le tassement progressif des greffons éventuellement ajoutés au cours de la consolidation sans engendrer l'instabilité de position de la cupule.

A cet effet, l'invention a pour objet une cupule hémisphérique non cimentée et non vissée à implanter dans la cavité cotyloïdienne osseuse, caractérisée en ce qu'elle comporte une tige de guidage allongée faisant saillie de la surface externe de la cupule, non filetée sur toute son étendue extérieurement saillante et destinée à être reçue dans un trou percé dans ladite cavité sur une longueur suffisante pour permettre l'enfoncement de la tige dans celui-ci lors de la migration de la cupule dans le bassin au cours de la consolidation.

Contrairement aux cupules connues où les vis stabilisent une fois pour toutes la cupule dans le cotyle, dans l'invention la stabilité s'améliore par migration dirigée de la cupule dans la cavité.

Dans le cas où des greffons sont utilisés, la tige de guidage favorise la stabilité tout en permettant le tassement de ces derniers.

Selon une autre caractéristique de l'invention, la tige de guidage est inclinée par rapport à l'axe de la cupule d'un angle compris entre environ 20 et 40° et de préférence 30°, de façon que cette tige soit implantée dans le cotyle selon l'axe des forces physiologiques, la tige étant ainsi alignée avec la résultante des forces exercées par la tête du fémur sur le toit du cotyle.

Cette orientation de la tige contribue à la fois à la stabilité de la cupule et permet l'implantation de la tige dans les parties saines du bassin, car à la verticale de cette force résultante, le bassin présente des travées osseuses en qualité, quantité et densité osseuses satisfaisantes.

Selon encore une autre caractéristique de l'invention, la tige précitée est lisse, c'est-à-dire non grenaillée comme l'étaient les vis des cupules de l'art antérieur en vue de la repousse osseuse, car cela gênerait la migration de la cupule.

Dans une variante de réalisation, la tige précitée peut être amovible et par exemple filetée à sa base pour venir se visser dans un taraudage correspondant pratiqué à travers la paroi de la cupule de reprise. La cupule est alors modulaire et peut être utilisée sans la tige en chirurgie de première intention si le chirurgien l'estime bon.

Dans ce cas, un bouchon fileté extérieurement est vissé dans ce taraudage, à la place de la tige, pour éviter le fluage de PET.

La cupule peut comporter en outre des pointes, par exemple trois pointes, coaxiales à la cupule et en saillie de sa surface externe pour l'ancrage primaire immédiat.

Ces pointes ainsi que la surface externe de la cupule sont de préférence grenaillées.

Le taraudage précité est de préférence localisé à égale distance de deux pointes voisines.

Selon une autre caractéristique de l'invention, la cupule présente sous son bord équatorial plusieurs pattes, par exemple quatre pattes, pour l'accrochage du noyau ou insert dans la cupule.

L'invention vise également une prothèse cotyloïdienne comportant la cupule précitée et un insert, par exemple en plastique tel que polyéthylène, à insérer dans celle-ci.

L'invention sera mieux comprise, et d'autres buts, caractéristiques, détails et avantages de celle-ci apparaîtront plus clairement au cours de la description explicative qui va suivre d'un mode de réalisation particulier actuellement préféré de l'invention, donné à titre d'exemple illustratif et non limitatif, en référence aux dessins schématiques annexés, dans lesquels :

La figure 1 est une vue éclatée montrant l'assemblage de la cupule de l'invention.

La figure 2 est une vue partiellement arrachée suivant la flèche II d'un détail encerclé sur la figure 1.

La figure 3 est une vue de dessous de la cupule de l'invention.

La figure 4 est une vue de dessus de la cupule de la figure 3.

La figure 5 est une vue en coupe suivant la ligne V-V de la cupule de la figure 4.

La figure 6 est une vue de côté, partiellement en coupe, d'un bouchon fileté à visser dans la cupule de l'invention.

Suivant l'exemple de réalisation représenté sur les dessins, la cupule 1 de l'invention présente une surface externe hémisphérique 1a et une surface interne cylindrosphérique 1b (voir figure 5).

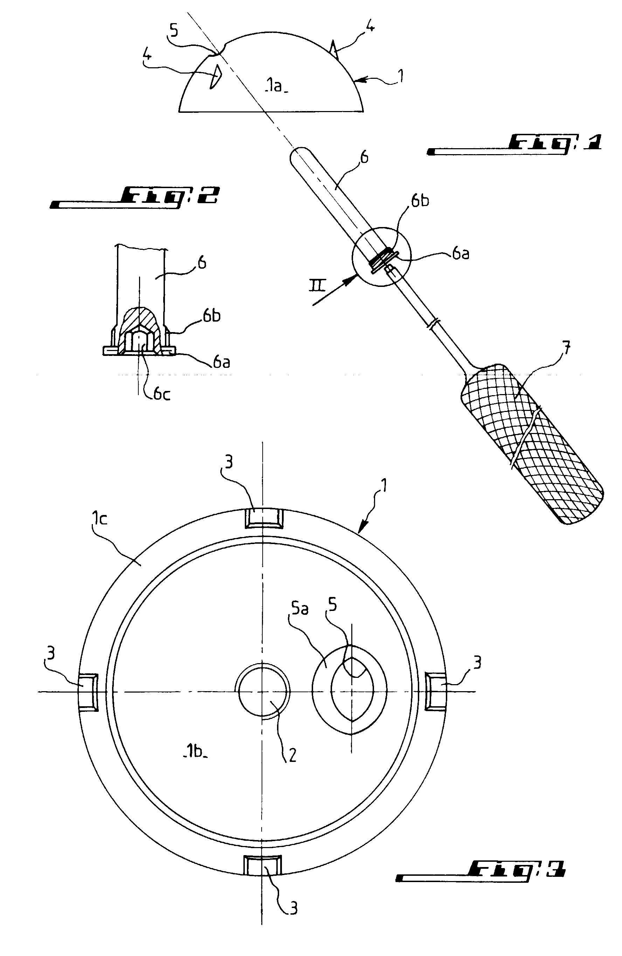

Un taraudage 2 est prévu au niveau du pôle de la cupule 1 coaxialement à l'axe A de celle-ci, pour le vissage par exemple d'un impacteur de cotyle ancillaire (non représenté). Mais on peut prévoir une cupule sans ce trou central pour éviter le fluage de PET dans le cotyle (voir figure 1).

Sous le bord équatorial 1c de la cupule 1 font axialement saillie quatre pattes 3 régulièrement réparties circonférentiellement pour l'accrochage sur un noyau en polyéthylène (non représenté) de la prothèse.

Trois pointes 4 font saillie de la surface externe la de la cupule 1 selon l'axe A et sont régulièrement réparties sur un parallèle de la cupule.

Bien entendu, on peut modifier le nombre, la répartition et l'orientation de ces pointes sans sortir du cadre de l'invention.

La cupule 1 comporte un autre taraudage 5 dont l'axe B fait un angle θ compris entre 20 et 40° et de préférence égal à 30° par rapport à l'axe A précité.

Un lamage 5a coaxial au taraudage 5 est formé sur la partie sphérique de la surface cylindrosphérique interne 1b de la cupule 1, pour recevoir la tête plate 6a d'une tige allongée 6.

La tige 6 comporte à sa base une portion filetée 6b venant se visser dans le taraudage 5 précité de façon que cette partie filetée 6b et la tête 6a soient complètement escamotées dans la paroi de la cupule 1 et logées respectivement dans le taraudage 5 et le lamage 5a associés.

La partie restante de la tige 6 qui est extérieurement saillante de la cupule 1 en position assemblée, est non filetée et lisse pour guider de manière coulissante la migration linéaire de la cupule 1 dans le cotyle au cours du tassement des greffons lors de la consolidation.

On voit sur la figure 1 que le sommet de la tige 6 est arrondi.

Les faces en regard du lamage 5a et de la tête plate 6a peuvent être avantageusement striées ou crantées pour bloquer en rotation la tige lorsqu'elle est vissée dans la cupule et éviter ainsi tout dévissage accidentel avant le montage du noyau.

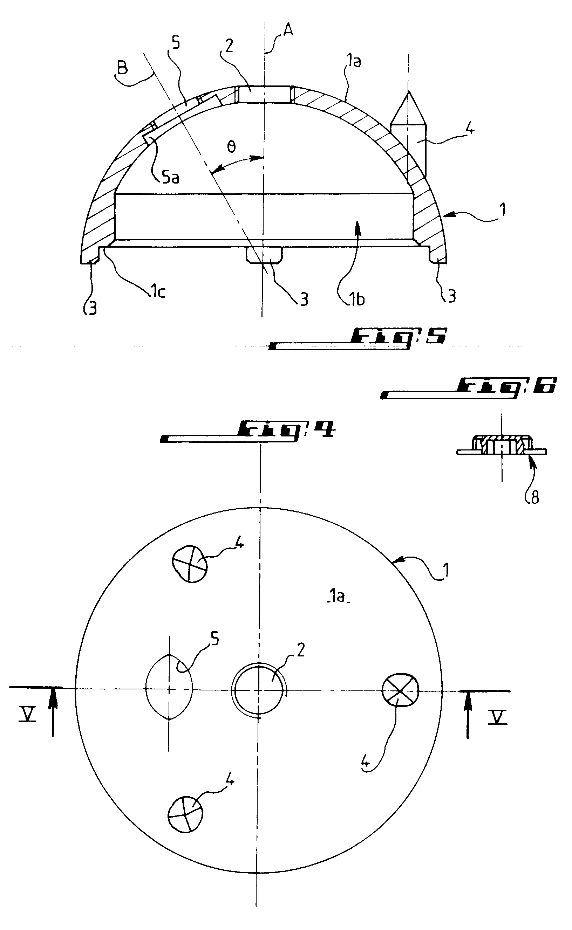

On voit sur la figure 2 qu'un trou héxagonal borgne 6c est percé dans la face opposée de la tête 6a pour permettre le vissage/dévissage de la tige 6 avec un tournevis ancillaire adapté 7.

A titre d'exemple, la longueur de la tige peut être comprise dans l'intervalle 25-65 mm, alors que les pointes précitées ont une hauteur de l'ordre de 10 mm pour un diamètre de cupule compris entre environ 46 mm et 68 mm.

On a représenté sur la figure 6, un bouchon 8 de forme analogue à la base de la tige amovible 6 précitée, pour venir obturer le taraudage 5 lorsque la cupule n'est pas utilisée avec la tige 6 ou le taraudage 2, afin d'éviter le fluage de plastique à travers ces derniers vers la cavité osseuse, pouvant entraîner une lyse osseuse par réaction à corps étranger.

Bien que l'invention ait été décrite en liaison avec un mode de réalisation particulier, il est bien évident qu'elle n'y est nullement limitée et qu'elle comprend tous les équivalents techniques des moyens décrits et leurs combinaisons si celles-ci entrent dans le cadre des revendications qui suivent.

## Revendications

1. Cupule hémisphérique non vissée (1) à implanter sans ciment dans la cavité cotyloïdienne osseuse, comportant une tige allongée (6) faisant saillie de la surface externe (1a) de la cupule, caractérisée en ce que ladite tige est non filetée sur toute son étendue extérieurement saillante et sert à guider de manière coulissante la migration linéaire de la cupule (1) dans le cotyle au cours de la consolidation, ladite tige ayant une longueur adaptée pour permettre l'enfoncement de celle-ci dans le trou correspondant percé dans la cavité cotyloïdienne lors de la migration de la cupule.

2. Cupule selon la revendication 1, caractérisé en ce que la tige de guidage (6) est inclinée par rapport à l'axe (A) de la cupule (1) d'un angle (θ) compris entre environ 20 et 40° et de préférence 30°, de façon que cette tige soit implantée dans le cotyle selon l'axe des charges physiologiques.

3. Cupule selon la revendication 1 ou 2, caractérisée en ce que la tige précitée (6) est lisse.

4. Cupule selon l'une des revendications précédentes, caractérisée en ce que la tige précitée (6) est amovible, rendant la cupule modulaire.

5. Cupule selon la revendication 4, caractérisée en ce que la tige (6) est filetée à sa base (6b) pour venir se visser dans un taraudage correspondant (5) pratiqué à travers la paroi de la cupule (1).

6. Cupule selon la revendication 5, caractérisée en ce qu'elle comporte un bouchon fileté (8) apte à venir se visser dans le taraudage (5) précité à la place de la tige de guidage (6).

7. Cupule selon l'une des revendications précédentes, caractérisée en ce qu'elle comporte en outre des pointes (3) coaxiales à la cupule (1) et en saillie de sa surface externe (1a) pour l'ancrage primaire immédiat.

8. Cupule selon la revendication 7, caractérisée en ce que les pointes sont au nombre de trois et le taraudage précité (5) est localisé à égale distance de deux pointes voisines.

9. Cupule selon l'une des revendications précédentes, caractérisée en ce qu'elle présente sous son bord équatorial (1c) plusieurs pattes (3) pour l'accrochage de l'insert dans la cupule.

10. Prothèse cotyloïdienne comportant la cupule selon l'une des revendications précédentes et un insert, par exemple en plastique tel que polyéthylène, à insérer dans celle-ci.
